(19) Europäisches Patentamt
European Patent Office
Office européen des brevets

(11) EP 1 673 480 B1

(12) **EUROPEAN PATENT SPECIFICATION**

(45) Date of publication and mention of the grant of the patent:
**14.04.2010 Bulletin 2010/15**

(21) Application number: **04816118.6**

(22) Date of filing: **09.09.2004**

(51) Int Cl.:
*C12Q 1/70* (2006.01) *G01N 33/00* (2006.01)
*C07H 21/02* (2006.01) *C12Q 1/68* (2006.01)
*C08F 2/48* (2006.01) *G03F 7/028* (2006.01)

(86) International application number:
**PCT/US2004/029733**

(87) International publication number:
**WO 2005/024386 (17.03.2005 Gazette 2005/11)**

# (54) USE OF PHOTOPOLYMERIZATION FOR AMPLIFICATION AND DETECTION OF A MOLECULAR RECOGNITION EVENT

VERWENDUNG DER PHOTOPOLYMERISIERUNG ZUR AMPLIFIKATION UND ZUM NACHWEIS EINES MOLEKULAREN ERKENNUNGSEREIGNISSES

UTILISATION DE LA PHOTOPOLYMERISATION POUR L'AMPLIFICATION ET LA DETECTION D'EVENEMENTS DE RECONNAISSANCE MOLECULAIRE

(84) Designated Contracting States:
**AT BE BG CH CY CZ DE DK EE ES FI FR GB GR HU IE IT LI LU MC NL PL PT RO SE SI SK TR**

(30) Priority: **09.09.2003 US 501755 P**

(43) Date of publication of application:
**28.06.2006 Bulletin 2006/26**

(73) Proprietor: **The Regents of the University of Colorado,**
**A Body Corporate**
**Denver, CO 80203 (US)**

(72) Inventors:
- **ROWLEN, Kathy, L.**
  **Boulder, CO 80503 (US)**
- **BIRKS, John, W.**
  **Boulder, CO 80303 (US)**
- **BOWMAN, Christopher**
  **Boulder, CO 80301 (US)**
- **SIKES, Hadley**
  **Cambridge, MA 02140 (US)**

(74) Representative: **O'Connell, Maura et al**
**FRKelly**
**4 Mount Charles**
**Belfast, Northern Ireland**
**BT7 1NZ (GB)**

(56) References cited:
**EP-A- 0 383 126** **US-A- 4 749 647**
**US-A- 5 449 602** **US-A- 5 637 460**
**US-B2- 6 806 047**

- **GAYLORD BRENT S ET AL: "DNA detection using water-soluble conjugated polymers and peptide nucleic acid probes" PROCEEDINGS OF THE NATIONAL ACADEMY OF SCIENCES OF USA, NATIONAL ACADEMY OF SCIENCE, WASHINGTON, DC, US, vol. 99, no. 17, 20 August 2002 (2002-08-20), pages 10954-10957, XP002406316 ISSN: 0027-8424**
- **COLLINS M L ET AL: "A branched DNA signal amplification assay for quantification of nucleic acid targets below 100 molecules/ml" NUCLEIC ACIDS RESEARCH, OXFORD UNIVERSITY PRESS, SURREY, GB, vol. 25, no. 15, 1997, pages 2979-2984, XP002208495 ISSN: 0305-1048**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 673 480 B1

## Description

BACKGROUND OF THE INVENTION

This invention is in the field of detection of molecular recognition events, in particular use of photopolymerization for amplification and detection of these events.

A variety of methods exist for detection of molecular recognition events. Detection of molecular recognition events such as DNA hybridization, antibody-antigen interactions, and protein-protein interactions becomes increasingly difficult as the number of recognition events to be detected decreases. Of particular interest are molecular recognition events between a target and a probe.

One approach to the problem is to increase the number of recognition events taking place. For example, polymerase chain reaction (PCR) increases the number of copies of DNA or RNA to be detected. Other molecular biology techniques which increase the number of copies of DNA or RNA to be detected include reverse transcription polymerase chain reaction (RT-PCR), strand displacement amplification, and Eberwine linear amplification.

Another approach is to amplify the signal due to each molecular recognition event. For example, DNA detection methods based on oligonucleotide-modified particles have been reported (U.S. Patents 6,740,491, 6,777,186, 6,773,884, 6,767,702, 6,759,199, 6,750,016, 6,730,269, 6,720,411, 6,720,147, 6,709,825, 6,682,895, 6,673,548, 6,667,122, 6,645,721, 6,610,491, 6,582,921, 6,506,564, 6,495,324, 6,417,340 and 6,361,944 and Park, S.-J. et al, 2002, Science, 295,5559, 1503-1506). U.S. Patent 6,602,669 relates to silver staining nanoparticles.

DNA detection methods based on branched DNA have also been reported (U.S. Patents 5,681,702, 5,597,909, 5,580,731, 5,359,100, 5,124,246, 5,545,730, 5,594,117, 5,571,670, 5,594,118, 5,681,697, 5,591,584, 5,571,670, 5,624,802, 5,635,352, and 5,591,584. The branched DNA assay is a solution phase assay that involves a number of probe oligonucleotides that bind to multiple sites on the target viral RNA. Detection is possible because each hybridization event is accompanied by the binding of a fluorophore (Kern, D., Collins, M., Fultz, T., Detmer, J., Hamren, S., Peterkin, J., Sheridan, P., Urdea, M., White, R., Yeghiazarian, T., Todd, J. (1996) "An Enhanced-sensitivity Branched-DNA Assay for Quantification of Human Immunodeficiency Virus Type 1 RNA in Plasma" Journal of Clinical Microbiology 34: 3196-3203). The synthetic effort required for this assay is relatively large: multiple probes are designed for each RNA of interest, and the assay depends on the binding of these probes to multiple preamplifier and amplifier molecules that also must be designed and synthesized.

Dendrimer-based DNA detection methods have also been reported (U.S. Patents 5,710,264, 5,175,270, 5,487,973, 5,484,904 and Stears, R. et al., 2000, Physiol. Genomics 3: 93-99). Dendrimers are complexes of partially double-stranded oligonucleotides, which form stable, spherical structures with a determined number of free ends. Specificity of the dendrimer detection is accomplished through specific binding of a capture oligonucleotide on a free arm of the dendrimer. Other arms of the dendrimer are labeled for detection. This method does not require enzymes and can produce amplification of 300 - 400.

Tyramide signal amplification is reported in U.S. Patents 6,593,100 and 6,372,937.

Rolling circle amplification has been described in the scientific literature (Baner et al. (1998) Nuc. Acids Res. 26:5073-5078; Barany, F. (1991) Proc. Natl. Acad. Sci. USA 88:189-193; Lizardi et al. (1998), Nat. Genet. 19:225-232; Zhang et al., Gene 211:277 (1998); and Daubendiek et al., Nature Biotech. 15:273 (1997)). Rolling circle amplification is capable of detecting as few as 150 molecules bound to a microarray (Nallur, G., Luo, C., Fang, L., Cooley, S., Dave, V., Lambert, J., Kukanskis, K., Kingsmore, S., Lasken, R., Schweitzer, B. (2001) "Signal Amplification by Rolling Circle Amplification on DNA Microarrays" Nucleic Acids Research 29:E118). The main drawback to RCA is the necessity of DNA polymerase.

Ligase chain reaction is reported in U.S. Patents 5,185,243 and 5,573,907.

Cycling probe technology is reported in U.S. Patents 5,011,769, 5,403,711, 5,660,988, and 4,876,187.

Microfabricated disposable DNA sensors based on enzymatic amplification electrochemical detection was reported by Xu et al. (Xu et al., 2001, Electoanalysis, 13(10), 882-887).

Surface initiated polymerization from surface confined initiators has been reported. Biesalski et al. report poly (methyl methacrylate) brushes grown in situ by free radical polymerization from a azo-initiator monolayer covalently bound to the surface (Biesalski, M. et al., (1999), J. Chem. Phys., 111(15), 7029). Surface initiated polymerization for amplification of patterned self-assembled monolayers by surface-initiated ring opening polymerization (Husemann, M. et al., Agnewandte Chemie Int. Ed. (1999), 38(5) 647-649) and atom transfer radical polymerization (Shah, R.R. et al., (2000), Macromolecules, 33, 597-605) has been also reported. EP-A-0 383 126 discloses a detection method where acrylamide monomers are polymerized into a detectable polymer in the presence at a photocatalyst.

DNA microarrays, or biochips, represent promising technology for accurate and relatively rapid pathogen identification (Wang, D., Coscoy, L., Zylberberg, M., Avila, P.C., Boushey, H.A., Ganem, D., DeRisi, J.L. (2002) "Microarray Based Detection and Genotyping of Viral Pathogens," PNAS, 99(24), 15687-15692). Anthony et al. recently demonstrated rapid identification of 10 different bacteria in blood cultures using a BioChip (Anthony, R.M., Brown, T.J., French, G.L.

(2000) "Rapid Diagnosis of Bacteremia by Universal Amplification of 23S Ribosomal DNA Followed by Hybridization to an Oligonucleotide Array" Journal of Clinical Microbiology 38:781-788). The microarray assay was conducted in ~ 4 hrs. The approach utilized universal primers for PCR amplification of the variable region of bacterial 23s ribosomal DNA, and a 3 x 10 array of 30 unique capture sequences. This work demonstrates an important aspect of BioChip platforms - the capability to screen for multiple pathogens simultaneously. DeRisi and co-workers demonstrated a "virus chip" that contained sequences for hundreds of viruses, including many that cause respiratory illness (Wang et al., 2002). This chip proved useful in identifying the corona virus associated with SARS (Risberg, E. (2003) "Gene Chip Helps identify Cause of Mystery Illness," USA Today (6/18/2003)). Evans and co-workers have demonstrated that a DNA microarray could be used for typing and sub-typing human influenza A and B viruses (Li, J., Chen, S., & Evans, D.H. (2001) "Typing and Subtyping Influenza Virus Using DNA Microarrays and Multiplex Reverse Transcriptase PCR" Journal of Clinical Microbiology 39:696-704). In both the DeRisi and Evans work PCR technology was used to amplify the genetic material for capture and relatively expensive fluorescent labels (~$50 in labels per chip) were used to generate signals from positive spots.

There remains a need in the art for relatively inexpensive labeling and signal amplification methods for molecular recognition events which do not require the use of enzymes for amplification. These methods would be useful in combination with DNA microarrays.

SUMMARY OF THE INVENTION

In an embodiment, the invention provides methods to detect molecular recognition events, in particular a relatively small number of molecular recognition events. The methods of the invention are based on amplification of the signal due to each molecular recognition event, rather than amplification of the number of molecular recognition events taking place. The present invention can replace PCR and RT-PCR techniques for microarray applications as a means to achieve acceptable signals.

In general, the methods of the invention can be used to generate and amplify a signal due to many types of molecular recognition events that can be described by the following equation:

$$A + B + In \rightarrow A\text{-}B\text{-}In \qquad \text{(Eqn 1)}$$

Where A and B are the species of interest that undergo molecular recognition and In is a photoinitiator. A is the probe species and B is the target species. For a microarray, the probe A is attached to the substrate. The target species, B, and/or the photoinitiator may comprise a linking group which allows selective binding of the photoinitiator to the target or the A-B complex. As an example, the target species may comprise biotin and the initiator avidin.

When the initiator comprises a linking group, Equation 1 may also be written as.

$$A + B + C\text{-}In \rightarrow A\text{-}B\text{-}C\text{-}In \qquad \text{(Eqn. 2)}$$

where C comprises an entity which allows selective binding of the photoinitiator to the target or the A-B complex.

The amplification scheme relies on the large number of propagation events that occur for each initiation event. Depending on the specific polymerization system used (light intensity, initiator concentration, monomer formulation, temperature, etc.), each initiator can lead to the polymerization of as many as $10^2$ - $10^6$ monomer units. Thus, each single molecular recognition event has the opportunity to be amplified by the polymerization of up to $10^6$ monomers, each of which may be fluorescent or enable detection of its presence through one of a variety of means. In an embodiment, a fluorescent polymer, a magnetic polymer, a radioactive polymer or an electrically conducting polymer can form the basis of detection and amplification. In another embodiment, a polymer gel swollen with a fluorescent solution, a magnetic solution, a radioactive solution or an electrically conducting solution can form the basis of detection and amplification.

In an embodiment, the invention provides a method for amplifying a molecular recognition interaction between a target and a probe comprising the steps of:

a) contacting the target with the probe under conditions effective to form a target-probe complex;
b) removing target not complexed with the probe;
c) contacting the target-probe complex with a photoinitiator label under conditions effective to attach the photoinitiator label to the target-probe complex;
d) removing photoinitiator label not attached to the target-probe complex;

e) contacting the photoinitiator-labeled target-probe complex with a polymer precursor;
f) exposing the photoinitiator-labeled target-probe complex and the polymer precursor to light, thereby forming a polymer; and
g) detecting the polymer formed, thereby detecting an amplified target-probe interaction.

In another embodiment, the invention provides methods for identification of a target species based on its molecular interaction with an array of different probe species, each probe species being attached to a solid substrate at known locations. In the methods of the invention, if the target species undergoes a molecular recognition reaction with a probe, the probe will be labeled with a polymer. Detection of the polymer-labeled probes allows identification of which probes have undergone the molecular recognition reaction and therefore identification of the target.

In an embodiment, the invention provides a method for identifying a target comprising the steps of

a) providing a probe array comprising a plurality of different probes, wherein the probes are attached to a solid substrate at known locations;
b) contacting the probe array with the target under conditions effective to form a target-probe complex;
c) removing target not complexed with the probe;
d) contacting the target-probe complex with a photoinitiator label under conditions effective to attach the label to the target-probe complex;
e) removing photoinitiator label not attached to the target-probe complex;
f) contacting the photoinitiator-labeled target-probe complex with a polymer precursor;
g) exposing the photoinitiator-labeled target-probe complex and the polymer precursor to light, thereby forming a polymer; and
h) detecting the polymer formed, wherein the polymer location indicates the probe which forms a target-probe complex with the target, thereby identifying the target.

The methods of the invention can be used to identify target species using DNA microarrays, also commonly referred to as DNA chips or BioChips, to provide the probe array. In an embodiment, the DNA microarray may be an array which allows identification and strain analysis for one or more genera of influenza virus.

In an embodiment, the methods of the invention provide sufficient amplification that molecular recognition can be detected using a relatively inexpensive microarray reader or scanner which may not have the highest instrument sensitivity or resolution.

BRIEF SUMMARY OF THE FIGURES

Figure 1 schematically illustrates some of the steps in detection and amplification of hybridization using photopolymerization.

Figure 2 illustrates an alternate two-step hybridization scheme for detection and amplification.

Figure 3 illustrates fluorescence detection of macroinitiators on a biotin array.

Figure 4 illustrates an image of a FluChip developed using photopolymerization for signal enhancement.

DETAILED DESCRIPTION OF THE INVENTION

Figure 1 is a conceptual diagram of how photopolymerization of a fluorescent monomer M is used to generate and amplify a signal from a single captured genetic target on a DNA microarray. Figure 1 shows addition of biotin (2) to the target oligonucleotide (10) to form a biotinylated target nucleotide (12). The biotinylated target nucleotide (12) is hybridized to a complementary probe (20), forming a target-probe complex (30) on the surface of the microarray (40). The microarray shown in Figure 1 contains a biotin-labeled probe (22) which acts as a positive control. After hybridization, the microarray is exposed to a photoinitiator label (50), initiator-functionalized avidin, which interacts with the biotinylated target oligonucleotide (12) and control probe (22) to form an initiator-labeled target-probe complex (35) and an initiator-labeled positive control probe (25). The microarray surface is then exposed to a fluorescent monomer (M) under the appropriate initiation conditions. In the presence of light and a fluorescent monomer (represented by M in Figure 1), a polymerization reaction occurs from sites on the surface where targets have been captured by the probe DNA, forming a polymer-labeled target-probe complex (37) and a polymer-labeled positive control probe (27). In Figure 1, the polymer

label is denoted by (60). Ideally, since initiators are not bound to sites where hybridizaton has not occurred, polymerization does not occur from those sites. For brevity, Figure 1 omits several steps which are typically used in the process, including removal of uncomplexed target material prior to exposure of the microarray to initiator functionalized avidin, removal of initiator functionalized avidin not attached to the target-probe complex, and removal of unpolymerized monomer prior to detection.

Although Figure 1 illustrates hybridization of complementary DNA to a DNA microarray as a specific example, the detection and amplification scheme generalizes to many other types of molecular recognition events. Agents capable of participating in molecular recognition events include, but are not limited to, agonists and antagonists for cell membrane receptors, toxins and venoms, viral epitopes, hormones (e.g., opiates, steroids, etc.), hormone receptors, peptides, enzymes, enzyme substrates, substrate analogs, transition state analogs, cofactors, drugs, proteins, and antibodies, cell membrane receptors, monoclonal antibodies and antisera reactive with specific antigenic determinants (such as on viruses, cells or other materials), drugs, polynucleotides, nucleic acids, peptides, cofactors, lectins, sugars, polysaccharides, cells, cellular membranes, and organelles. In different embodiments, the detection and amplification scheme can be used to detect and amplify the molecular recognition interaction between nucleic acids, an antibody and an antigen, and a first and a second protein. Microarrays can be used to detect hybridization as well as protein-protein interactions, protein drug binding, and enzymatic catalysis (Schena, M., "Microarray Analysis, (2003) John Wiley & Sons, New Jersey, p. 153). As used herein, molecular recognition interactions are those in which the probe recognizes and selectively binds a target, resulting in a target-probe complex. Molecular recognition interactions also involve the formation of noncovalent bonds between the two species. The binding occurs between specific regions of atoms (molecular domains) on the probe species which have the characteristic of binding or attaching specifically to unique molecular domains on specific target species. Molecular recognition interactions can also involve responsiveness of one species to another based on the reciprocal fit of a portion of their molecular shapes.

The target and probe are two species of interest which undergo molecular recognition. The target may also be referred to as a ligand. The probe may also be referred to as a receptor. In an embodiment, at least some characteristics of the probe are known. In an embodiment, the probe is an oligonucleotide whose sequence is known or partially known. In other embodiment, the sequence of the probe may not be known, but it is known to be complementary to a possible target species. Typically, the probe will be selected so that it is capable of selected recognition with the known or suspected identity of the target. In some cases a single probe can be used to detect the presence of a target. In other cases more than one probe will be necessary to detect the presence of or identify a target.

In order for molecular interaction between the target and the probe to identify the target, the molecular interaction between the target and the probe must be sufficiently specific. For hybridization, the selectivity is a measure of the specificity of the molecular recognition event. "Selectivity" or "hybridization selectivity" is the ratio of the amount of hybridization (*i.e.*, number of second nucleic acids hybridized) of fully complementary hybrids to partially complementary hybrids, based on the relative thermodynamic stability of the two complexes. For the purpose of this definition it is presumed that this ratio is reflected as an ensemble average of individual molecular binding events. Selectivity is typically expressed as the ratio of the amount of hybridization of fully complementary hybrids to hybrids having one base pair mismatches in sequence. Selectivity is a function of many variables, including, but not limited to,: temperature, ionic strength, pH, immobilization density, nucleic acid length, the chemical nature of the substrate surface and the presence of polyelectrolytes and/or other oligomers immobilized on the substrate or otherwise associated with the immobilised film.

For hybridization, the homology of the target and probe molecules influences whether hybridization occurs. Cross-hybridization can occur if the sequence identity between the target and the probe is greater than or equal to about 70% (Schena, M., "Microarray Analysis, (2003) John Wiley & Sons, New Jersey, p. 151).

In an embodiment, either the target or the probe is a nucleic acid. In an embodiment, both the target and the probe are a single stranded nucleic acid. In an embodiment, the probe is an oligonucleotide, a relatively short chain of single-stranded DNA or RNA. "Nucleic acid" includes DNA and RNA, whether single or double stranded. The term is also intended to include a strand that is a mixture of nucleic acids and nucleic acid analogs and/or nucleotide analogs, or that is made entirely of nucleic acid analogs and/or nucleotide analogs and that may be conjugated to a linker molecule. "Nucleic acid analogue" refers to modified nucleic acids or species unrelated to nucleic acids that are capable of providing selective binding to nucleic acids or other nucleic acid analogues. As used herein, the term "nucleotide analogues" includes nucleic acids where the internucleotide phosphodiester bond of DNA or RNA is modified to enhance bio-stability of the oligomer and "tune" the selectivity/specificity for target molecules (Uhlmann, et al., (1990), Angew. Chem. Int. Ed. Eng., 90: 543; Goodchild, (1990), J. Bioconjugate Chem., I: 165; Englisch et al., (1991), Angew, Chem. Int. Ed. Eng., 30: 613). Such modifications may include and are not limited to phosphorothioates, phosphorodithioates, phosphotriesters, phosphoramidates or methylphosphonates. The 2'-O-methyl, allyl and 2'-deoxy-2'-fluoro RNA analogs, when incorporated into an oligomer show increased biostability and stabilization of the RNA/DNA duplex (Lesnik et al., (1993), Biochemistry, 32: 7832). As used herein, the term "nucleic acid analogues" also include alpha anomers ($\alpha$-DNA), L-DNA (mirror image DNA), 2'-5' linked RNA, branched DNA/RNA or chimeras of natural DNA or RNA and the above-modified nucleic acids. For the purposes of the present invention, any nucleic acid containing a "nucleotide analogue" shall be

considered as a nucleic acid analogue. Backbone replaced nucleic acid analogues can also be adapted to for use as immobilised selective moieties of the present invention. For purposes of the present invention, the peptide nucleic acids (PNAs) (Nielsen et al., (1993), Anti-Cancer Drug Design, 8: 53; Engels et al., (1992), Angew, Chem. Int. Ed. Eng., 31: 1008) and carbamate-bridged morpholino-type oligonucleotide analogs (Burger, D.R., (1993), J. Clinical Immunoassay, 16: 224; Uhlmann, et al., (1993), Methods in Molecular Biology, 20,. "Protocols for Oligonucleotides and Analogs," ed. Sudhir Agarwal, Humana Press, NJ, U.S.A., pp. 335-389) are also embraced by the term "nucleic acid analogues". Both exhibit sequence-specific binding to DNA with the resulting duplexes being more thermally stable than the natural DNA/DNA duplex. Other backbone-replaced nucleic acids are well known to those skilled in the art and can also be used in the present invention (See. e.g., Uhlmann et al., (1993), Methods in Molecular Biology, 20, "Protocols for Oligonucleotides and Analogs," ed. Sudhir Agrawal, Humana Press, NJ, U.S.A., pp. 335).

More generally, the probe and/or target can be an oligomer. "Oligomer" refers to a polymer that consists of two or more monomers that are not necessarily identical. Oligomers include, without limitation, nucleic acids (which include nucleic acid analogs as defined above), oligoelectrolytes, hydrocarbon based compounds, dendrimers, nucleic acid analogues, polypeptides, oligopeptides, polyethers, oligoethers any or all of which may be immobilized to a substrate . Oligomers can be immobilized to a substrate surface directly or *via* a linker molecule.

In an embodiment, the probe is DNA. The DNA may be genomic DNA or cloned DNA. The DNA may be complementary DNA (cDNA), in which case the target may be messenger RNA (mRNA). The DNA may also be an Expressed Sequence Tag (EST) or a Bacterial Artificial Chromosome (BAC). For use in hybridization microarrays, double-stranded probes are denatured prior to hybridization, effectively resulting in single-stranded probes.

DNA microarrays are known to the art and commercially available. The general structure of a DNA microarray is a well defined array of spots on an optically flat surface, each of which contains a layer of relatively short strands of DNA. As referred to herein, microarrays have a spot size less than about 1.0 mm. In most hybridization experiments, 15-25 nucleotide sequences are the minimum oligonucleotide probe length (Schena, M., "Microarray Analysis, (2003) John Wiley & Sons, New Jersey, p. 8). The substrate is generally flat glass primed with an organosilane that contains an aldehyde functional group. The aldehyde groups facilitate covalent bond formation to biomolecules with free primary amines via Schiff base interactions. After reaction the chip is cured to form a very stable array ready for hybridization.

Protein microarrays are also known to the art and some are commercially available. The general structure of protein microarrays can be similar to that of DNA microarrays, except that array spots can contain antibodies (in particular monoclonal antibodies), antigens, recombinant proteins, or peptides. For accurate measurement of binding events, surface-bound proteins must be correctly folded and fully functional (Constans, A. 2004, The Scientist, 18(15) 42). To reduce protein unfolding, the proteins can be protected by use of stabilizing buffers and/or relatively high protein concentrations (Schena, M., "Microarray Analysis, (2003) John Wiley & Sons, New Jersey, p. 154). To avoid the protein folding problem, the functional domains of interest can be arrayed rather than the whole protein, forming domain-based arrays (Constans, 2004, ibid).

In an embodiment, the target is genetic material from influenza A, B, or C. Influenza is an orthomyxovirus with three genera, types A, B, and C. The types are distinguished by the nucleoprotein antigenicity (Dimmock, N.J., Easton, A.J., Leppard, K.N. (2001) "Introduction to Modern Virology" 5th edition, Blackwell Science Ltd., London). Influenza A and B each contain 8 segments of negative sense ssRNA. Type A viruses can also be divided into antigenic sub-types on the basis of two viral surface glycoproteins, hemagglutinin (HA) and neuraminidase (NA). There are currently 15 identified HA sub-types (designated H1 through H15) and 9 NA sub-types (N1 through N9) all of which can be found in wild aquatic birds (Lamb, R.A. & Krug, R.M., (1996) "Orthomyxoviridae: The Viruses and their Replication, in Fields Virology", B.N. Fields, D.M. Knipe, and P.M. Howley, Editors. Lippincott-Raven: Hagerstown). Of the 135 possible combinations of HA and NA, only four (H1N1, H1N2, H2N2, and H3N2) have widely circulated in the human population since the virus was first isolated in 1933. The two most common sub-types of influenza A currently circulating in the human population are H3N2 and H1N1. LI et al. describe a DNA microarray whose probes were multiple fragments of the hemagglutinin, neuraminidase, and matrix protein genes. (Li, J. et al., (2001), J. Clinical Microbio., 39(2), 696-704).

For probes bound to a substrate using aldehyde attachment chemistry, the substrate may be treated with an agent to reduce the remaining aldehydes prior to contacting the probe with the target. One suitable reducing agent is sodium borohydride $NaBH_4$. Such a treatment can decrease the amount of reaction between the monomer and the aldehyde coating on the glass, thus decreasing the amount of background signal during the detection step.

Prior to contacting the target with the probe, the target may be biotinylated to allow later attachment of at least one initiator via biotin-avidin interaction. In an embodiment, photobiotinylation reagents (Pierce, Quanta Biodesign) can be used to biotin-label the target.

In an embodiment, the target may be contacted with the photoinitiator label prior to contacting the target with the probe, so long as use of a photoinitiator-labeled target does not substantially limit its participation in the desired molecular recognition event. In an embodiment, the invention provides a method for amplifying a molecular recognition interaction between a target and a probe comprising the steps of contacting a photoinitiator-labeled target with a probe under conditions effective to form a photoinitiator-labeled target-probe complex, removing target not complexed with the

probe, contacting the photoinitiator-labeled target-probe complex with a polymer precursor, exposing the photoinitiator-labeled target-probe complex and the polymer precursor to light, thereby forming a polymer ,and detecting the polymer formed, thereby detecting an amplified target-probe interaction.

The probe is contacted with a solution comprising the target under conditions effective to form a target-probe complex. The conditions effective to form a target-probe complex depend on the target and probe species. For ssDNA or RNA targets binding to ssDNA probes, suitable hybridization conditions have been described in the scientific literature. In an embodiment, it is sufficient to contact a solution comprising the target with the probe for about 2 hours at about 42 °C. In an embodiment, this solution also comprises an agent, such as a crowding agent, to limit nonspecific interactions. With reference to nucleic acid interactions, a crowding agent is an agent that interrupts nonspecific adsorption between nucleic acids that are not complementary. Formamide is one such agent to limit nonspecific interactions (Stahl, D. A., and R. Amann. 1991. Development and application of nucleic acid probes, p.205-248. In E. Stackebrandt and M. Goodfellow (ed.), Nucleic acid techniques in bacterial systematics. John Wiley & Sons Ltd., Chichester, United Kingdom). Nonspecific interactions can also be limited by applying a blocking agent to the microarray prior to contacting the target with the probe. Suitable blocking agents are known to the art and include, but are not limited to BSA , nonfat milk, and sodium borohydride. Detergents such as sodium lauroyl sarcosine or sodium dodecyl sulfate can also be added to aldehyde surface hybridization reactions to reduce background (Schena, M., "Microarray Analysis, (2003) John Wiley & Sons, New Jersey, p. 117). The target solution may also be contacted with the probe at higher temperatures in order to limit nonspecific interactions.

After the target is contacted with the probe, targets which have not formed target-probe complexes are removed. The unbound targets can be removed through rinsing. Water or an aqueous solution may be used for rinsing away unbound targets.

If the initiator is to be attached through biotin-avidin interaction, a blocking agent can be applied to the microarray to limit nonspecific interaction of avidin. Suitable blocking agents are known to the art and include, but are not limited to, BSA and nonfat milk. In an embodiment array is incubated with the blocking agent for approximately 20 minutes at about room temperature.

In an embodiment, the target-probe complex is contacted with a photoinitiator label under conditions effective to attach the photoinitiator label to the target probe complex. In an embodiment, the photoinitiator label comprises avidin or streptavidin and at least one photoinitiator. In an embodiment, a plurality of photoinitiators are attached to the avidin or streptavidin to form a polymeric photoinitiator label. In another embodiment, a plurality of photoinitiators and avidin or streptavidin are attached to a polymer. If the target has been biotin-labeled, interaction between the avidin or streptavidin and the biotin can attach the photoinitiator label to the target, and thus to the target-probe complex. Information on avidin-biotin interaction is provided in Wilcheck, M., (a) Bayer, E.A. Eds. (1990) "Avidin-biotin technology" Methods in Enzymology 184. In an embodiment, the biotin-labeled target-probe complex is contacted with a solution comprising the photoinitiator label for about 20 minutes at room temperature.

Another embodiment suitable for hybridization molecular recognition events is schematically illustrated in Figure 2. In this embodiment, the photoinitiator label (50) comprises a single strand of DNA attached to at least one photoinitiator (In). The photoinitiator can be attached to the ssDNA through biotinylation of the DNA followed by interaction with avidin or streptavidin with at least one photoinitiator attached. In another embodiment, photoinitiators are coupled directly to the end of the oligonucleotide label sequences. Oligonucleotides with 5' amine modifications can be purchased and the reaction conditions in Scheme 2 (EDC coupling) used to form a peptide bond between this amine and the carboxylic acid group of the initiator. The product can be purified by HPLC. During the photopolymerization reaction, the target anchors the initiator, through the label sequence, to the microarray spot. If target viral RNA will not tolerate the presence of the fluorescent monomer and UV light, it is possible to connect the label sequence to the probe oligonucleotide via a treatment with ligase prior to exposure to the photopolymerization reaction conditions. Another method that avoids the use of an enzyme is to place pendant photocrosslinkable groups on the probe oligonucleotide and the label sequence. If, however, the polymerization reaction is fast when compared with the timescale of diffusion, these steps will not be necessary even if the target genetic material detaches from the capture strand.

A number of photoinitiators are known to the art. Photoinitiators that are useful in the invention include those that can be activated with light and initiate polymerization of the polymer precursor. In an embodiment, the photoinitiator is water soluble. Commercially available photoinitiators, for example Irgacure 2959 (Ciba), can be modified to improve their water solubility. In an embodiment, the photoinitiator is a radical photoinitiator. In another embodiment, the photoinitiator is a cationic photoinitiator. In another embodiment, the photoinitiator comprises a carboxylic acid functional group. The photoinitiator is selected to be compatible with the wavelengths of light supplied.

Photoinitiators include azobisisobutyronitrile, peroxides, phenones, ethers, quinones, acids, formates. Cationic initiators include aryldiazonium, diaryliodonium, and triarylsulfonium salts. In an embodiment, the photoinitiator is selected from the group consisting of Rose Bengal (Aldrich), Darocur 2959 (2-hydroxy-1-(4-(hydroxyethoxy)phenyl)-2-methyl-1-propanone, D2959, Ciba-Geigy), Irgacure 651 (2,2-dimethoxy-2-phenylacetophenone, I651, DMPA, Ciba-Geigy), Irgacure 184 (1-hydroxycyclohexyl phenyl ketone, I184, Ciba-Geigy), Irgacure 907 (2-methyl-1-(4-(methylthio)phenyl)-2-(4-morpholinyl)-1-propanone, 1907, Ciba-Geigy), Camphorquinone (CQ, Aldrich), isopropyl thioxanthone (quantacure ITX, Great Lakes Fine Chemicals LTD., Cheshire, England). CQ is typically used in conjunction with an amine such as ethyl 4-N,N-dimethylaminobenzoate (4EDMAB, Aldrich) or triethanolamine (TEA, Aldrich) to initiate polymerization.

Photoinitiator molecules can be attached to avidin or streptavidin by modification of avidin or streptavidin lysine residues. For photoinitiators having a carboxylic acid functional group, the carboxylic functional group of the photoinitiator can be coupled to the amine of the lysine residue in the presence of a coupling agent. The result is the formation of a peptide bond between the initiator and the protein.

In another embodiment, a polymeric photoinitiator label is formed. Such a polymeric photoinitiator label can be formed from a polymer which can be coupled with both the photoinitiator and avidin or streptavidin. In an embodiment, the polymer comprises carboxylic acid groups and amide groups. In an embodiment, the polymeric photoinitiator comprises sufficient photoinitiators so that it may be regarded as a macroinitiator (having many initiators present on a single molecule). In an embodiment, the use of a macroinitiator can increase the initiator concentration by a factor of between about 10 to about 100.

After contact of the photoinitiator label with the target-probe complex, unattached photoinitiator is removed. Unattached photoinitiator may be removed by rinsing with water or an aqueous solution.

The photoinitiator-labeled target-probe complex is contacted with a solution comprising a polymer precursor. As used herein a "polymer precursor" means a molecule or portion thereof which can be polymerized to form a polymer or copolymer. Polymer precursors include any substance that contains an unsaturated moiety or other functionality that can be used in chain polymerization, or other moiety that may be polymerized in other ways. Such precursors include monomers and oligomers. In an embodiment, the solution further comprises a solvent for the polymer precursor. In an embodiment, the solvent is aqueous.

The solution may further comprise oxygen inhibition agents and/or cross-linking agents. In an embodiment, the oxygen inhibition agent is a multithiol (Bhanu, V.A. & Kishore, K. (1991) Role of Oxygen in Polymerization Reactions, Chemical Reviews 91: 99-117). The amount of oxygen inhibition agent should not be so much that polymerization occurs in the bulk of the solution rather than from the surface. A crosslinking agent can stabilize the polymer that is formed and improve the amplification factor (Hacioglu B., Berchtold K.A., Lovell L.G., Nie J., & Bowman C.N. (2002) Polymerization Kinetics of HEMA/DEGDMA: using Changes in initiation and Chain Transfer Rates to Explore the Effects of Chain-Length-Dependent Termination. Biomaterials 23:4057-4064). Finally, a small amount of inhibitor can be added to the formulation to limit background polymerization caused by impurities and trace radicals formed by absorption by molecules other than the initiator.

In different embodiments, the polymer precursor is a photopolymerizable monomer capable of forming a fluorescent polymer, a magnetic polymer, a radioactive polymer or an electrically conducting polymer. In an embodiment, the polymer precursor is water soluble. In an embodiment, the polymer precursor is a photopolymerizable fluorescent methacrylate monomer. When the polymer precursor is fluorescent, the fluorophore may absorb the light used in the photopolymerization process. To compensate, the exposure time of the polymer precursor to the light and/or the light intensity can be adjusted.

In another embodiment, the polymer precursor is capable of forming a polymer gel. In an embodiment, the gel is covalently crosslinked and a cross-linking agent is added to the polymer precursor containing solution. In another embodiment, the gel is noncovalently crosslinked. In an embodiment, the polymer gel formed is not substantially fluorescent, magnetic, radioactive, or electrically conducting. Instead, detection occurs through absorption of a fluorescent, magnetic, radioactive, or electrically conducting solution by the gel.

In an embodiment, the polymer gel is a hydrogel. The term "hydrogel" refers to a class of polymeric materials which are extensively swollen in an aqueous medium, but which do not dissolve in water. In general terms, hydrogels are prepared by polymerization of a hydrophilic monomer under conditions where the polymer becomes cross-linked in a three dimensional matrix sufficient to gel the solution. The hydrogel may be natural or synthetic. A wide variety of hydrogel-forming compositions are known to the art. In an embodiment, the monomer used to form the hydrogel is selected from the group consisting of acrylates, methacrylates, acrylamides, methacrylamides, cyclic lactams and monomers with ionic functionality. Monomers with ionic functionality include methacrylate, methacrylamide, and styrene based monomers with acidic or basic functionality. In an embodiment, the monomer used to form the hydrogel is an acrylate or methacrylate. In another embodiment, the hydrogel-forming monomer is selected from the group consisting of polysaccharides and proteins. Polysaccharides capable of forming hydrogels include alginate, chitin, chitosan, cellulose, oligopeptides, and hyalauric acids. Proteins capable of forming hydrogels include albumin and gelatin. In an embodiment, the monomer is hydroxyl ethyl acrylate (HEA).

The photoinitiator-labeled target-probe complex and polymer precursor are exposed to light, thereby forming a polymer. Photopolymerization occurs when polymer precursor solutions are exposed to light of sufficient power and of a wavelength capable of initiating polymerization. The wavelengths and power of light useful to initiate polymerization depends on the initiator used. Light used in the invention includes any wavelength and power capable of initiating polymerization. Preferred wavelengths of light include ultraviolet or visible. Any suitable source may be used, including

laser sources. The source may be broadband or narrowband, or a combination. The light source may provide continuous or pulsed light during the process. Both the length of time the systemis exposed to UV light and the intensity of the UV light can be varied to determine the ideal reaction conditions. For fluorescence detection, the exposure time and light intensity can be varied to obtain maximal fluorescence signal from spots on a microarray and minimal fluorescence signal from the background.

In an embodiment, after polymerization, unpolymerized polymer precursor is removed. The unpolymerized polymer precursor can be removed by rinsing, for example by rinsing with water or an aqueous solution. The unpolymerized polymer precursor need not be removed if formation of the polymer is to be detected by its refractive index or by other means that would not be interfered with by the presence of the unpolymerized polymer precursors.

If the hydrogel polymer is not substantially fluorescent, magnetic, radioactive, or electrically conducting, the hydrogel is contacted with a detectable solution which is fluorescent, magnetic, radioactive, or electrically conducting so that the hydrogel absorbs a sufficient quantity of the detectable solution. After the detectable solution is absorbed into the hydrogel, the excess solution is removed before detection.

In an embodiment, the polymer formed is detected by fluorescence, magnetic, radioactive or electrical detection methods as are known to the art. If the probes are part of a DNA microarray, a commercially available microarray scanner and/or imager can be used to detect polymer formation. DNA microarray scanners and/or imagers are commercially available that can detect fluorescent or radioisotopic labels.

Analysis of polymer formation can allow identification of the target. Methods of design and analysis of DNA microrrays for identification of target molecules are known to the art (Vernet, G. (2002) "DNA-Chip Technology and Infectious Diseases" Virus Research 82:65-71). Similar methods, appropriately modified, can be applied to other types of microarrays and molecular recognition events.

As used herein, "comprising" is synonymous with "including," "containing," or "characterized by," and is inclusive or open-ended and does not exclude additional, unrecited elements or method steps. As used herein, "consisting of' excludes any element, step, or ingredient not specified in the claim element. As used herein, "consisting essentially of" does not exclude materials or steps that do not materially affect the basic and novel characteristics of the claim. Any recitation herein of the term "comprising", particularly in a description of components of a composition or in a description of elements of a device, is understood to encompass those compositions and methods consisting essentially of and consisting of the recited components or elements. The invention illustratively described herein suitably may be practiced in the absence of any element or elements, limitation or limitations which is not specifically disclosed herein.

The terms and expressions which have been employed are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

In general the terms and phrases used herein have their art-recognized meaning, which can be found by reference to standard texts, journal references and contexts known to those skilled in the art. The above definitions are provided to clarify their specific use in the context of the invention.

All patents and publications mentioned in the specification are indicative of the levels of skill of those skilled in the art to which the invention pertains.

**Example 1: Synthesis of a Water Soluble Initiator**

***Scheme 1.***

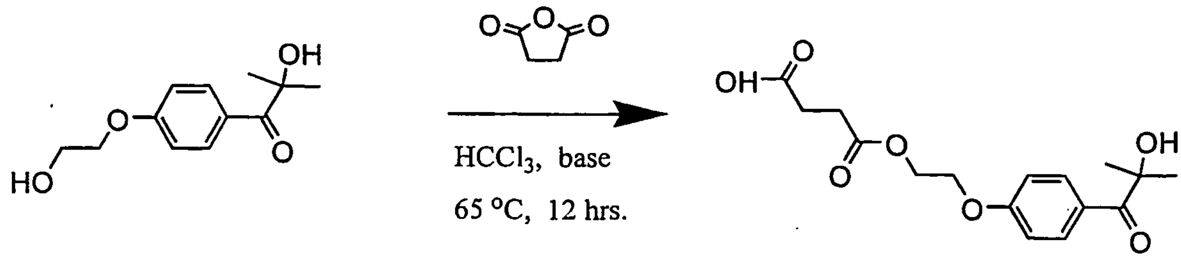


As shown in Scheme 1, synthesis of a water soluble photoinitiator (preferred for compatibility with a BioChip) was achieved by starting with commercially available Irgacure 2959 (left most structure, Ciba Specialty Chemicals (http://www.cibasc.com)). Irgacure 2959 was dissolved/suspended in chloroform along with succinic anhydride and a catalytic amount of 4-dimethylaminopyridine. The solution was refluxed, with stirring, for 12 hours at 65°C. In both chloroform and water, the product was soluble while the starting materials were sparingly soluble. The product structure was verified by NMR and shown to function as a photoinitiator by monitoring the double bond conversion of an acrylate monomer using time-resolved FTIR.

**Example 2: Functionalization of Avidin with Photoinitiator**

***Scheme 2.***

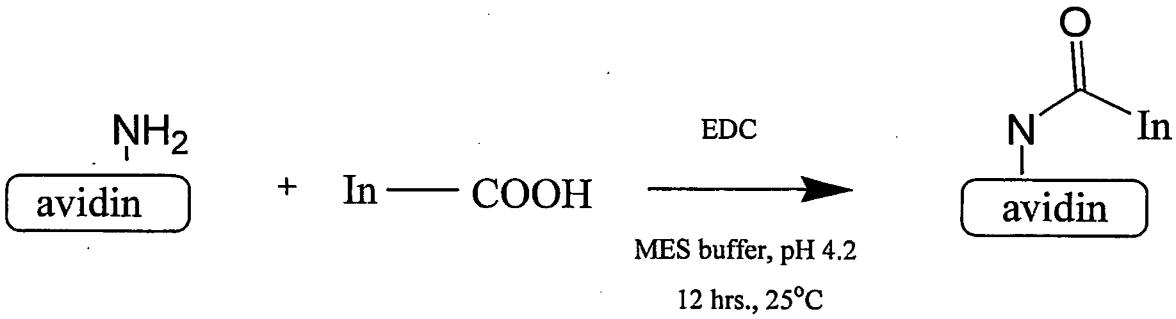


Avidin is often labeled with dye molecules by modification of its many lysine residues(for example, Pierce Biotechnology sells a kit for this purpose). These types of modifications do not disrupt avidin's ability to bind to biotin (Wilbur, D.S.; Hamlin, D.K.; Buhler, K.R.; Pathare, P.M.; Vessella, R.L.; Stayton, P.S.; To, R. (1998) "Streptavidin in antibody pretargeting. 2. Evaluation of methods for decreasing localization of streptavidin to kidney while retaining its tumor binding capacity" Bioconjugate Chemistry 9: 322-330). Here, as shown in Scheme 2, the lysine residues have been modified with a photoinitiator (rather than a dye) by coupling the carboxylic acid functional group of the photoinitiator to the amine of the lysine residue. The result is formation of a peptide bond between the initiator and the protein. Avidin and an excess of the initiator (the product in Scheme 1, represented by In in Scheme 2) were dissolved in an acid aqueous buffered solution in the presence of the water-soluble coupling agent 1-ethyl-3-(3-dimethylaminopropyl) carbodiimide (EDC) (Hermanson, G.T. (1996) Bioconjugate Techniques San Diego, CA: Academic Press. p. 435). The reaction proceeded at room temperature for 12 hours, and the product was collected by ultracentrifugation through a 3,000 MW cutoff filter. Biotin binding capabilities were verified using the HABA assay (Wilcheck, M., (b) Bayer, E.A. Eds. (1990) "Protein biotinylation" Methods in Enzymology 184: 138-160).

**Example 3: Synthesis of a Polymer Labeled with Photoinitiator and Streptavidin**

Scheme 3:

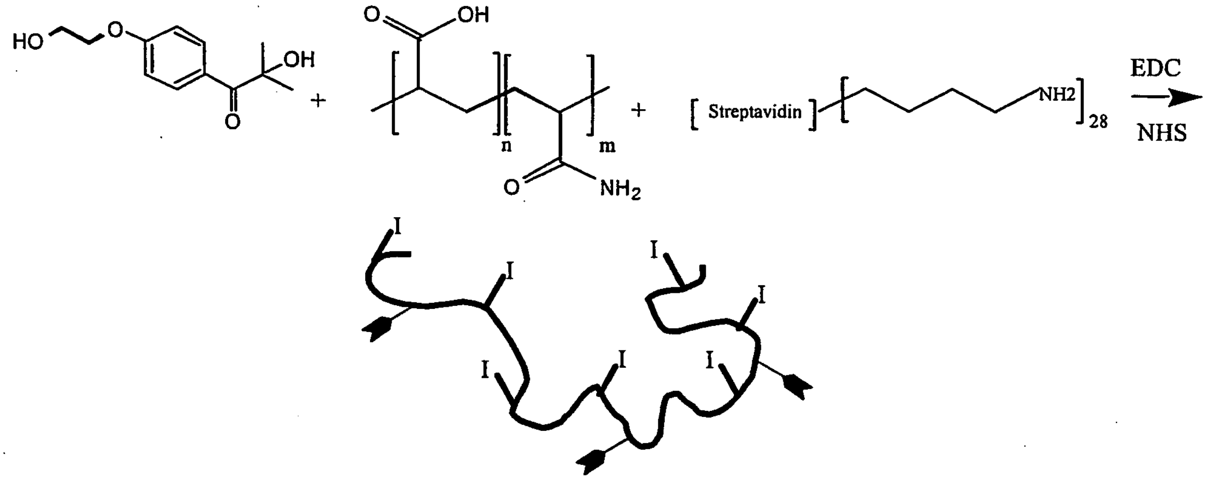


Scheme 3 illustrates the formation of a polymer labeled with Irgacure 2959 photoinitiator (denoted by I) and streptavidin (denoted by

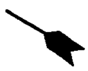

). The reaction takes place in the presence of EDC and N-hydroxysuccinimide (NHS) at room temperature for about two hours.

**Example 4: Macroinitiator Synthesis**

In the event that a label sequence containing a single initiator does not provide a high enough level of amplification with the short irradiation times necessary to minimize background fluorescence, macroinitiators, in which many initiators are present on a single molecule, can be used. Synthesis of a macroinitiator can be achieved through a living (or controlled) radical polymerization method prior to utilization on the microarrays (Kamigaito M., Ando T., & Sawamoto M. (2001) "Metal-Catalyzed Living Radical polymerization. Chemical Reviews 101: 3689-3746). Atom transfer radical polymerization (ATRP) schemes can be used to control the macroinitiator molecular weight, composition and architecture (block copolymers, branching, etc.) (Matyjaszewski, K. & Jianhui Xia, J. (2001) Atom Transfer Radical Polymerization Chemical Reviews 101: 2921 -2990).

***Scheme 4**. Reaction for generation of macroinitiator.*

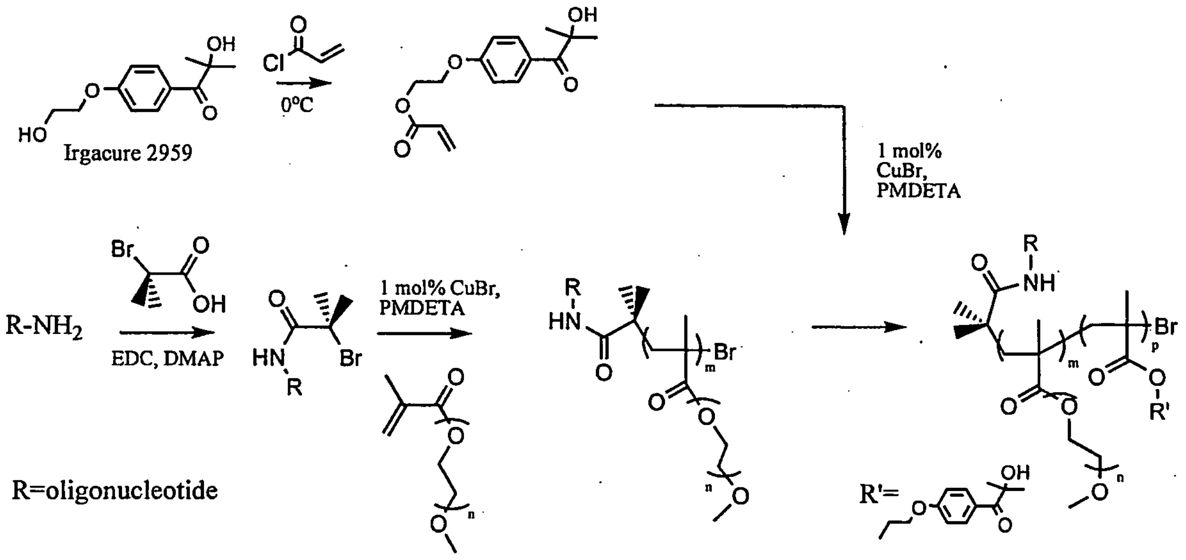


Macroinitiator synthesis can be performed, as presented in Scheme 4, by starting with an oligonucleotide terminated in an amine group. The amine terminus is functionalized with an ATRP initiator, which initiates polymerization of the desired compounds. As many as three different monomers (an initiator, a spacer, and a photosensitizer) may need to be copolymerized. If necessary to improve hybridization to the probe oligonucleotide, a spacer molecule (for example (poly ethylene glycol methacrylate)) is used between the initiating block of the macroinitiator and the oligonucleotide part of the label sequence. The spacer may also be used for controlling solubility of the macroinitiator (by changing the hydrophobicity/hydrophilicity) and the macroinitiator molecular weight. Photosensitizing components are incorporated into the macroinitiator in systems where minimization of the background polymerization is necessary.

**Example 5: Synthesis and photopolymerization of a fluorescent monomer**

***Scheme 5.***

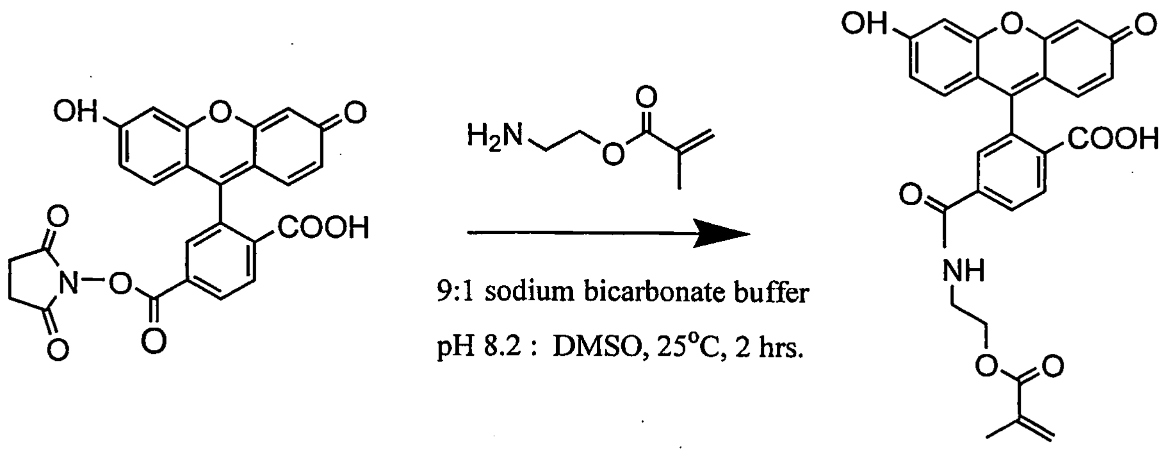


The N-hydroxy succinimide ester of fluorescein was purchased from Pierce Biotechnology (Rockford, IL) and 2 mg were dissolved in 100 μL of DMSO while 0.5 mg of 2-aminoethyl methacrylate was dissolved in 900 μL of sodium bicarbonate buffer, pH 8.2. The two solutions were combined and placed on a shaker for two hours. The solvent was

lyophilized off. The structures of the fluorescent monomer and the polymer that results from irradiating the monomer with UV light were verified by NMR.

The monomer can be polymerized by irradiating with 365 nm ultraviolet light for one minute.

**Example 6: Formation of a Hydrogel from a Polymer Labeled with Photoinitiator and Streptavidin and Detection of the Hydrogel Formed.**

The polymer of Example 3 was reacted with a microarray having biotin covalently bound to the microarray substrate. A hydroxyl ethyl acrylate monomer solution was placed in contact with the array by pipetting the solution into a HybriWell (Grace Biolabs) which covers the array. Sixty μl of an aqueous solution was used which contained hydroxyl ethyl acrylate, initiator (product of Scheme 1 and Scheme 3), cross-linking agent (ethylene glycol dimethacrylate, 3% by volume), and oxygen inhibition agent (mercaptoethanol, 5 x $10^{-4}$ % by volume).

The monomer was then photopolymerized to form a hydrogel by irradiating the array with 365 nm light for about 1 minute.

After polymerization, the microarray was rinsed with water and then a solution containing Rhodamine B, concentration 100 nM, was pipetted between a glass coverslip and the microarray. The microarray was exposed to the fluorophore containing solution for about 30 minutes. The microarray was then rinsed with water to remove excess fluorophore .

Figure 3A is an image of the array after polymerization. The grid of reacted biotin spots (100) are bright and indicate areas of formation of hydrogel swollen with the fluorescent solution. The detector used was an Agilent Microarray Scanner (Fluorescence Ave: 2600 , Std: 1200 Background: 1200Signal to Noise: 2.2) Figure 3B shows negative controls spots (110) on the same array (Fluorescence Ave: 900, Std: 30, Background: 800, Signal to Noise: 1.1)

**Example 7: On-Chip Hybridization, Amplification, and Detection on a Flu Chip.**

On-chip signal amplification by photopolymerization was tested on a custom microarray designed to detect and subtype the influenza virus. As shown in Figure 4, the chip had a column of positive control spots (80), spots to capture RNA of influenza C (91), spots to capture RNA of influenza B (93), and spots to capture RNA of influenza A (95). There were three spots in each column. The sequence that is complementary to the positive control spots (but not to the other nine spots on the array) was purchased from Qiagen (Valencia, CA) with a 5' biotin modification. Published protocols were used to hybridize a 1 μM solution of this oligonucleotide to the microarray. The hybridization procedure was as follows:

Pre-hybridization

1. Boil $dH_2O$
2. While $dH_20$ is boiling, wash microarray in 0.1% SDS for 2-10 min on rocker. (0.5 ml 10% SDS in 50 ml total volume)
3. Transfer to 2X SSC for 2 min. (5 ml 20X in 50 ml total volume)
4. Immerse slide in boiling/hot (>95°C) water for 2-3 min.
5. Remove slide, blot on kimwipe.
6. Immerse slide in ice-cold ethanol bath for 2-5 min. Store EtOH at -20°C.
7. Remove slide, blot on kimwipe.
8. Centrifuge ethanol off of slide. (50 ml conical tube, 1000 rpm, 3 min)

Hybridization

1. Pipette 10 uL of water or buffer into the humidification slots, place microarray in hybridization cassette.
2. Hybridization solution:

   10 μL 50 micromolar oligo soln (PBS or Tris)
   10 μL 10X SSC,
   5 μL 1% Tween 20
   5μL 10 mM $MgCl_2$
   20μL$H_2O$

3. Pipette 7 μL of this solution between a coverslip covering the microarray and the slide.
4. Seal cassette, submerge in water bath (40-45°C) for at least 1.5-2 hrs.

Washing

1. Transfer microarray immediately into 1X SSC (2.5 ml 20X in 50 ml total) /0.1% SDS (0.5 ml 10% SDS in 50 ml total). 5 min.
2. Transfer microarray into 0.1X SSC (0.25 ml 20X in 50 ml)/ 0.1% SDS. 5 min.
3. Immerse briefly in 0.1X SSC to remove SDS.

Rinse with water, dry with $N_2$.

Following hybridization, 7 μL of a 1 mg/ml solution of BSA was pipetted between a glass coverslip and the microarray to block nonspecific binding. Following twenty minutes of incubation in a humid hybridization chamber, the array was rinsed with water. Subsequently, 7 μL of a 1 mg/ml initiator-functionalized avidin solution was pipetted between a glass coverslip and the microarray. After twenty minutes of incubation and brief rinsing with water, 7 μL of a saturated solution of the fluorescent monomer in water was pipetted between the glass coverslip and the microarray. The array was irradiated with 365 nm UV light for one minute, rinsed with water, and imaged using an Agilent (Wilmington, DE) microarray scanner. The excitation wavelength ($\lambda_{ex}$=532 nm) and collection wavelength (centered at 575 nm) were not optimal for the fluorescein derivative (product in Scheme 3). Figure 4 is the resulting image

In Figure 4, the lighter spots represent detected fluorescence. Though the background fluorescence remains relatively high despite the BSA treatment, the positive control spots (80) are definitely more fluorescent than the spots designed to capture the genetic material of influenza A, B and C (these spots effectively served as three different negative controls in this experiment). The faint gray vertical lines in Figure 4 are a well understood artifact. Glass slides were placed between the microarray and the UV light source to block high frequency UV light that is harmful to the fluorophore. The vertical lines arise from the seam between two glass slides that were used as a filter in this manner

Two other control experiments were performed. Without the BSA nonspecific binding blocking step, and all other steps held constant, the entire image yielded significant fluorescence, indicating nonspecific binding of the initiator. In addition, without the addition of avidin-functionalized initiator, when all other steps remained identical, none of the spots exhibited fluorescence yet the background was still high. These results suggest that the fluorescent spots in Figure 4 do indeed result from an interaction between the monomer and the selectively bound initiator. These tests, taken together, indicate that photopolymerization is clearly a viable means to obtain selective signal amplification directly on a DNA microarray

Without wishing to be bound by any specific theory, one possible cause of nonspecific binding is reaction of the fluorescent monomer with the aldehyde coating on the glass. To eliminate this source of nonspecific binding, alternative attachment chemistries are available, or the unreacted aldehydes outside of the oligonucleotide spots can be passivated or reduced. If macroinitiators containing larger numbers of initiators are used, the irradiation time can be reduced. To further reduce nonspecific binding, an inhibitor can be included. This technique will be effective if the number of initiation sites inside the hybridized spots (specific, due to the presence of many initiators) is much greater than the number of possible sites outside the spots (nonspecific, absorption and initiation occurring without initiator, and non-specific binding). False positives can be minimized by the use of macroinitiators, photosensitizers/inhibitors and optimal initiation time and light intensity, combined with judicious choice of probe and label sequence and steps to prevent non-specific binding of the initiator to the array

**Example 8: Quantification of Number of Fluors.**

The number of fluorescent molecules incorporated into the polymer and therefore bound to the microarray surface can be quantified. One test is to measure the shape and size of the resulting spot after polymerization is complete using either atomic force microscopy or profilometry. With this information and knowledge of the mass fraction of fluor within the mixture and the polymer density, it is possible to calculate relatively accurately the number of fluors within the polymer. This approach minimizes error due to any fluorescence quenching within the polymer. Quenching is quantified and minimized by conducting a study of fluorescence intensity as a function of mass percent fluor in the polymer. Due to the amorphous nature of the polymer, quenching is not expected to be significant.

**Claims**

**1.** A method for amplifying a molecular recognition interaction between a target and a probe comprising the steps of:

a. contacting the target with the probe under conditions effective to form a target-probe complex;
b. removing target not complexed with the probe;

c. contacting the target-probe complex with a photoinitiator label under conditions effective to attach the photoinitiator label to the target-probe complex, the photoinitiators label including a photoinitiator;
d. removing photoinitiator label not attached to the target-probe complex;
e. contacting the photoinitiator-labeled target-probe complex with a solution comprising a polymer precursor;
f. exposing the photoinitiator-labeled target-probe complex and the polymer precursor to light, thereby forming a polymer; and

detecting the polymer formed, thereby detecting an amplified target-probe interaction.

**2.** The method of claim 1, wherein the probe is attached to a solid substrate.

**3.** The method of claim 1, wherein the target and the probe are contacted to form a target-probe complex by contacting a probe array with the target, the probe array comprising a plurality of different probes attached to a solid substrate at known locations.

**4.** The method of claim 2 or 3, wherein nonspecific interactions between the substrate and the target are limited by prior application of a blocking agent to the substrate prior to step a) or by use of a crowding agent during step a).

**5.** The method of any of claims 2-4, wherein nonspecific interactions between the substrate and the photoinitiator label are limited by prior application of a blocking agent to the substrate prior to step c).

**6.** The method of any of claims 1-5, further comprising the step of removing unpolymerized polymer precursor prior to detecting polymer formation.

**7.** The method of any of claims 1-6, wherein the target comprises biotin, the photoinitiator label comprises avidin or streptavidin, and the photoinitiator label is attached to the target-probe complex by interaction between the biotin and the avidin or streptavidin.

**8.** The method of any of claims 1-7, wherein the target comprises single-stranded DNA (ssDNA) or RNA and the probe comprises ssDNA having a sequence complementary to at least a portion of the sequence of the target; wherein the target optionally comprises Influenza A, B, or C RNA; or wherein the target comprises one of an antibody or antigen and the probe comprises the other of an antibody or antigen; or wherein the target comprises a first protein, the probe comprises a second protein, and the first and second proteins are capable of molecular recognition.

**9.** The method of any of claims 1-8, wherein the photoinitiator is a radical photoinitiator and in step e) the photoinitiator-labeled target probe complex is contacted with the polymer precursor in the presence of an agent to control oxygen inhibition of radical polymerization.

**10.** The method of any of claims 1-9 wherein the photoinitiator label contains a plurality of photoinitiators; wherein the photoinitiator label is optionally a macroinitiator.

**11.** The method of any of claims 1 -10, wherein the target comprises biotin and the photoinitiator label is a polymeric photoinitiator label formed from a second polymer which is coupled with both the photoinitiator and avidin or streptavidin.

**12.** The method of any of claims 1-11, wherein the polymer is a fluorescent polymer, a magnetic polymer, a radioactive polymer, or an electrically conductive polymer.

**13.** The method of any of claims 1-12 wherein during step e) the photoinitiator-labeled target-probe complex is contacted with the polymer precursor solution in the presence of a cross-linking agent and the polymer formed in step f) is a hydrogel; the method optionally further comprising the steps of i) contacting the hydrogel with a solution comprising a fluorophore, thereby allowing absorption of the fluorophore solution by the hydrogel and ii) removing excess fluorophore solution, both steps being performed prior to step g).

**14.** A method for identifying a target comprising the steps of providing a probe array comprising a plurality of different probes, wherein the probes are attached to a solid substrate at known locations; and amplifying the molecular recognition interaction between the target and the probe of the array which forms a target-probe complex with the target by the method of any of claims 2-13, wherein the target and the probe are contacted by contacting the probe

array with the target and the location of the polymer formed indicates the probe which forms a target-probe complex with the target, thereby identifying the target.

**15.** The method of claim 14, wherein the probe array contains ssDNA, antigens, and proteins; wherein the probe array optionally contains ssDNA complementary to Influenza A, B, or C RNA, Influenza A, B, or C antigens, and proteins which capture proteins which form in the outside of Influenza A, B, or C viral particles.

**16.** The method of claim 14, wherein the exposing step comprises exposing the photoinitiator-labeled target-probe complex and the polymer precursor to light, thereby forming a polymer-labeled probe.

## Patentansprüche

**1.** Verfahren zum Amplifizieren einer molekularen Erkennungswechselwirkung zwischen einem Target und einer Sonde, umfassend die Schritte von:

a. Inkontaktbringen des Targets mit der Sonde unter Bedingungen, die zur Bildung eines Target-Sonden-Komplexes wirksam sind;
b. Entfernen des nicht mit der Sonde komplexierten Targets;
c. Inkontaktbringen des Target-Sonden-Komplexes mit einer Photoinitiator-Markierung unter Bedingungen, die zum Anbinden der Photoinitiator-Markierung an den Target-Sonden-Komplex wirksam sind, wobei die Photoinitiator-Markierung einen Photoinitiator einschließt;
d. Entfernen der nicht an den Target-Sonden-Komplex angebundenen Photoinitiator-Markierung;
e. Inkontaktbringen des Photoinitiator-markierten Target-Sonden-Komplexes mit einer Lösung, die einen Polymer-Präkursor umfasst;
f. Exponieren des Photoinitiator-markierten Target-Sonden-Komplexes und des Polymer-Präkursors gegenüber Licht, wodurch ein Polymer gebildet wird; und

Nachweisen des gebildeten Polymers, wodurch eine amplifizierte Target-Sonden-Wechselwirkung nachgewiesen wird.

**2.** Verfahren nach Anspruch 1, worin die Sonde an ein festes Substrat angebunden ist.

**3.** Verfahren nach Anspruch 1, worin das Target und die Sonde zum Bilden eines Target-Sonden-Komplexes durch Inkontkaktbringen eines Sonden-Arrays mit dem Target, wobei das Sonden-Array eine Vielzahl verschiedener Sonden umfasst, die an bekannten Stellen an ein festes Substrat angebunden sind, in Kontakt gebracht werden.

**4.** Verfahren nach Anspruch 2 oder 3, worin nicht spezifische Wechselwirkungen zwischen dem Substrat und dem Target durch vorherige Applikation eines Blockierungsmittels auf das Substrat vor Schritt a) oder durch Verwendung eines "Crowding"-Mittels während Schritt a) begrenzt sind.

**5.** Verfahren nach einem der Ansprüche 2-4, worin nicht spezifische Wechselwirkungen zwischen dem Substrat und der Photoinitiator-Markierung durch vorherige Applikation eines Blockierungsmittels auf das Substrat vor Schritt c) begrenzt sind.

**6.** Verfahren nach einem der Ansprüche 1-5, das ferner den Schritt des Entfernens von nicht polymerisiertem Polymer-Präkursor vor dem Nachweis der Polymerbildung umfasst.

**7.** Verfahren nach einem der Ansprüche 1-6, worin das Target Biotin umfasst, die Photoinitiator-Markierung Avidin oder Streptavidin umfasst und die Photoinitiator-Markierung an den Target-Sonden-Komplex durch Wechselwirkung zwischen dem Biotin und dem Avidin oder Streptavidin angebunden wird.

**8.** Verfahren nach einem der Ansprüche 1-7, worin das Target einsträngige DNA (ssDNA) oder RNA umfasst und die Sonde ssDNA mit einer Sequenz umfasst, die zu mindestens einem Anteil der Sequenz des Targets komplementär ist; worin das Target optional RNA von Influenza A, B oder C umfasst; oder worin das Target eines von einem Antikörper oder Antigen umfasst und die Sonde das andere von einem Antikörper oder Antigen umfasst; oder worin das Target ein erstes Protein umfasst, die Sonde ein zweites Protein umfasst und die ersten und zweiten Proteine zur molekularen Erkennung fähig sind.

**9.** Verfahren nach einem der Ansprüche 1-8, worin der Photoinitiator ein radikalischer Photoinitiator ist und der Photoinitiator-markierte Target-Sonden-Komplex in Schritt e) mit dem Polymer-Präkursor in Gegenwart eines Mittels zur Kontrolle der Sauerstoff-Inhibition der radikalischen Polymerisation in Kontakt gebracht wird.

**10.** Verfahren nach einem der Ansprüche 1-9, worin die Photoinitiator-Markierung eine Vielzahl von Photoinitiatoren enthält; worin die Photoinitiator-Markierung optional einen Makroinitiator darstellt.

**11.** Verfahren nach einem der Ansprüche 1-10, worin das Target Biotin umfasst und die Photoinitiator-Markierung eine polymere Photoinitiator-Markierung darstellt, die aus einem zweiten Polymer gebildet wird, das sowohl mit dem Photoinitiator als auch Avidin oder Streptavidin gekoppelt ist.

**12.** Verfahren nach einem der Ansprüche 1-11, worin das Polymer ein fluoreszierendes Polymer, ein magnetisches Polymer, ein radioaktives Polymer oder ein elektrisch leitendes Polymer ist.

**13.** Verfahren nach einem der Ansprüche 1-12, worin der Photoinitiator-markierte Target-Sonden-Komplex während Schritt e) mit der Polymer-Präkursorlösung in Gegenwart eines Vernetzungsmittels in Kontakt gebracht wird und das in Schritt f) gebildete Polymer ein Hydrogel darstellt; wobei das Verfahren optional ferner die folgenden Schritte umfasst: i) das Inkontaktbringen des Hydrogels mit einer einen Fluorophor umfassenden Lösung, wodurch die Absorption der Fluorophor-Lösung durch das Hydrogel erlaubt wird und ii) das Entfernen der überschüssigen Fluorophor-Lösung, wobei beide Schritte vor Schritt g) durchgeführt werden.

**14.** Verfahren zum Identifizieren eines Targets, das die folgenden Schritte umfasst: Bereitstellen eines Sonden-Arrays, umfassend eine Vielzahl verschiedener Sonden, worin die Sonden an ein festes Substrat an bekannten Stellen angebunden sind; und Amplifizieren der molekularen Erkennungswechselwirkung zwischen dem Target und der Sonde des Arrays, das einen Target-Sonden-Komplex mit dem Target mithilfe des Verfahrens nach einem der Ansprüche 2-13 bildet, worin das Target und die Sonde durch Inkontaktbringen des Sonden-Arrays mit dem Target in Kontakt gebracht werden und die Stelle des gebildeten Polymers die Sonde anzeigt, welche mit dem Target einen Target-Sonden-Komplex bildet, womit das Target identifiziert wird.

**15.** Verfahren nach Anspruch 14, worin das Sonden-Array ssDNA, Antigene und Proteine enthält; worin das Sonden-Array optional zur RNA von Influenza A, B oder C komplementäre ssDNA, Antigene von Influenza A, B oder C und Proteine, welche Proteine einfangen, die sich in der Außenseite von Viruspartikeln von Influenza A, B oder C bilden, enthält.

**16.** Verfahren nach Anspruch 14, worin der Expositionsschritt das Exponieren des Photoinitiator-markierten Target-Sonden-Komplexes und des Polymer-Präkursors gegenüber Licht umfasst, wodurch eine Polymer-markierte Sonde gebildet wird.

## Revendications

**1.** Méthode d’amplification d’une interaction de reconnaissance moléculaire entre une cible et une sonde, comprenant les étapes consistant à :

a. mettre en contact la cible et la sonde dans des conditions efficaces pour former un complexe cible-sonde ;
b. éliminer la cible non complexée avec la sonde ;
c. mettre en contact le complexe cible-sonde avec un marqueur photo-initiateur dans des conditions efficaces pour fixer le marqueur photo-initiateur au complexe cible-sonde, le marqueur photo-initiateur comprenant un photo-initiateur ;
d. éliminer le marqueur photo-initiateur non fixé au complexe cible-sonde ;
e. mettre en contact le complexe cible-sonde marqué par le photo-initiateur avec une solution comprenant un précurseur polymère ;
f. exposer le complexe cible-sonde marqué par le photo-initiateur et le précurseur polymère à de la lumière, formant ainsi un polymère ; et

détecter le polymère formé, détectant ainsi une interaction cible-sonde amplifiée.

**2.** Méthode selon la revendication 1, dans laquelle la sonde est fixée à un substrat solide.

3. Méthode selon la revendication 1, dans laquelle la cible et la sonde sont mises en contact afin de former un complexe cible-sonde par la mise en contact d'un réseau de sondes avec la cible, le réseau de sondes comprenant une pluralité de différentes sondes fixées à un substrat solide au niveau d'emplacements connus.

4. Méthode selon la revendication 2 ou 3, dans laquelle les interactions non spécifiques entre le substrat et la cible sont limitées par l'application préalable d'un agent bloquant au substrat avant l'étape a) ou par l'utilisation d'un agent d'entassement lors de l'étape a).

5. Méthode selon l'une quelconque des revendications 2-4, dans laquelle des interactions non spécifiques entre le substrat et le marqueur photo-initiateur sont limitées par l'application préalable d'un agent bloquant au substrat avant l'étape c).

6. Méthode selon l'une quelconque des revendications 1-5, comprenant en outre l'étape d'élimination du précurseur polymère non polymérisé préalablement à la détection de la formation de polymère.

7. Méthode selon l'une quelconque des revendications 1-6, dans laquelle la cible comprend de la biotine, le marqueur photo-initiateur comprend de l'avidine ou de la streptavidine, et le marqueur photo-initiateur est fixé au complexe cible-sonde par interaction entre la biotine et l'avidine ou la streptavidine.

8. Méthode selon l'une quelconque des revendications 1-7, dans laquelle la cible comprend de l'ADN simple brin (ADNss) ou de l'ARN et la sonde comprend de l'ADNss ayant une séquence complémentaire avec au moins une portion de la séquence de la cible ; dans laquelle la cible comprend éventuellement de l'ARN de Grippe A, B ou C ; ou dans laquelle la cible comprend l'un parmi un anticorps ou antigène et la sonde comprend l'autre parmi un anticorps ou antigène ; ou dans laquelle la cible comprend une première protéine, la sonde comprend une deuxième protéine, et les première et deuxième protéines sont aptes à la reconnaissance moléculaire.

9. Méthode selon l'une quelconque des revendications 1-8, dans laquelle le photo-initiateur est un photo-initiateur radicalaire et dans l'étape e), le complexe cible-sonde marqué par le photo-initiateur est mis en contact avec le précurseur polymère en présence d'un agent afin de contrôler l'inhibition par l'oxygène de la polymérisation radicalaire.

10. Méthode selon l'une quelconque des revendications 1-9, dans laquelle le marqueur photo-initiateur contient une pluralité de photo-initiateurs ; dans laquelle le marqueur photo-initiateur est éventuellement un macro-initiateur.

11. Méthode selon l'une quelconque des revendications 1-10, dans laquelle la cible comprend de la biotine et le marqueur photo-initiateur est un marqueur photo-initiateur polymère formé à partir d'un deuxième polymère qui est couplé avec le photo-initiateur ainsi qu'avec l'avidine ou la streptavidine.

12. Méthode selon l'une quelconque des revendications 1-11, dans laquelle le polymère est un polymère fluorescent, un polymère magnétique, un polymère radioactif, ou un polymère électriquement conducteur.

13. Méthode selon l'une quelconque des revendications 1-12, dans laquelle, lors de l'étape e), le complexe cible-sonde marqué par le photo-initiateur est mis en contact avec la solution de précurseur polymère en présence d'un agent de réticulation et le polymère formé dans l'étape f) est un hydrogel ; la méthode comprenant éventuellement en outre les étapes consistant à i) mettre en contact l'hydrogel avec une solution comprenant un fluorophore, permettant ainsi l'absorption de la solution de fluorophore par l'hydrogel et ii) éliminer l'excès de solution de fluorophore, les deux étapes étant réalisées avant l'étape g).

14. Méthode d'identification d'une cible, comprenant les étapes de fourniture d'un réseau de sondes comprenant une pluralité de sondes différentes, dans laquelle les sondes sont fixées à un substrat solide au niveau d'emplacements connus ; et l'amplification de l'interaction de reconnaissance moléculaire entre la cible et la sonde du réseau qui forme un complexe cible-sonde avec la cible par la méthode selon l'une quelconque des revendications 2-13, où la cible et la sonde sont mises en contact par la mise en contact du réseau de sondes avec la cible et l'emplacement du polymère formé indique la sonde qui forme un complexe cible-sonde avec la cible, identifiant ainsi la cible.

15. Méthode selon la revendication 14, dans laquelle le réseau de sondes contient de l'ADNss, des antigènes et des protéines ; dans laquelle le réseau de sondes contient éventuellement de l'ADNss complémentaire à l'ARN de Grippe A, B ou C, des antigènes de Grippe A, B ou C, et des protéines qui piègent des protéines se formant à

l'extérieur des particules virales de Grippe A, B ou C.

**16.** Méthode selon la revendication 14, dans laquelle l'étape d'exposition comprend l'exposition du complexe cible-sonde marqué par le photo-initiateur et du précurseur polymère à de la lumière, formant ainsi une sonde marquée par le polymère.

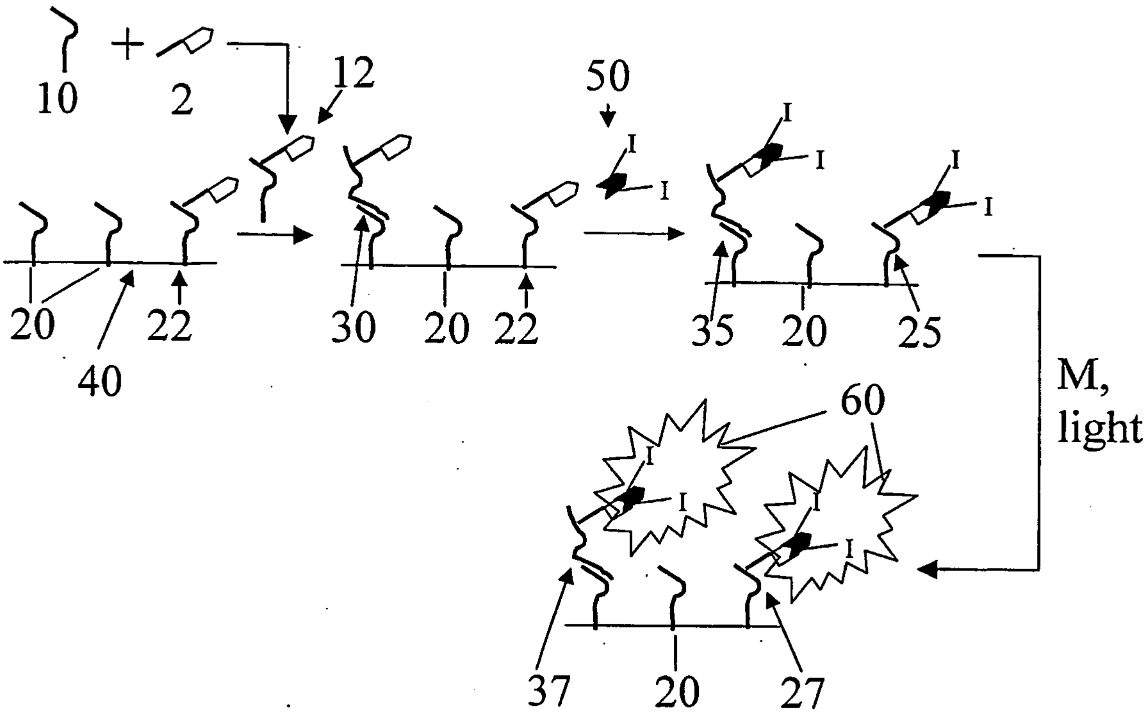


**Figure 1**

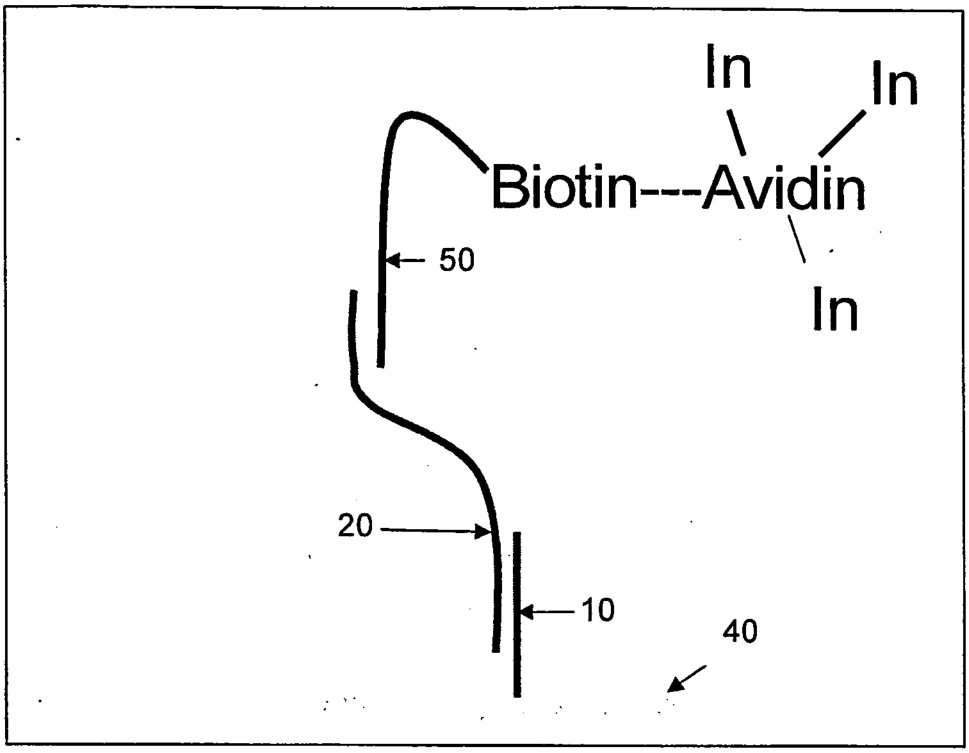


Figure 2

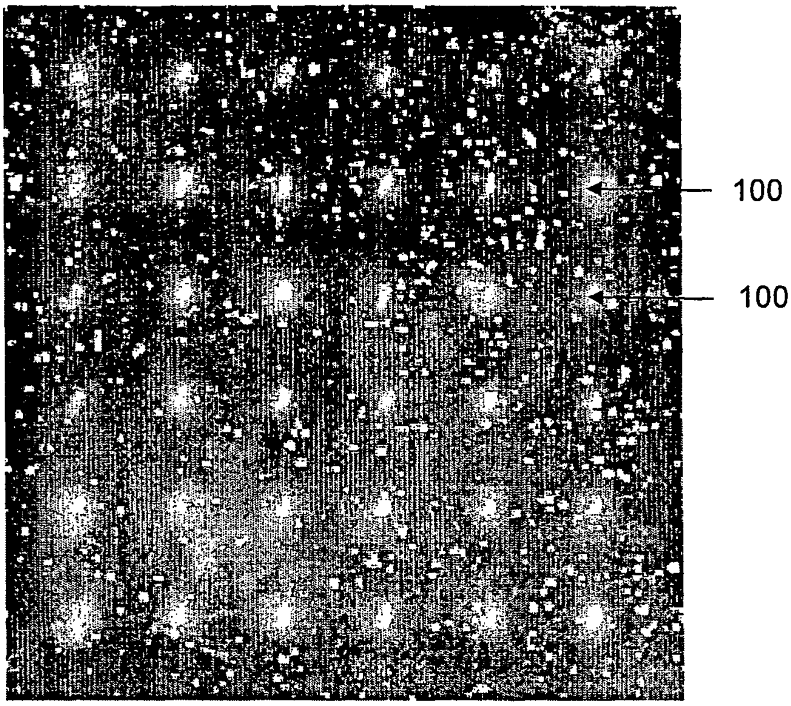


Figure 3A

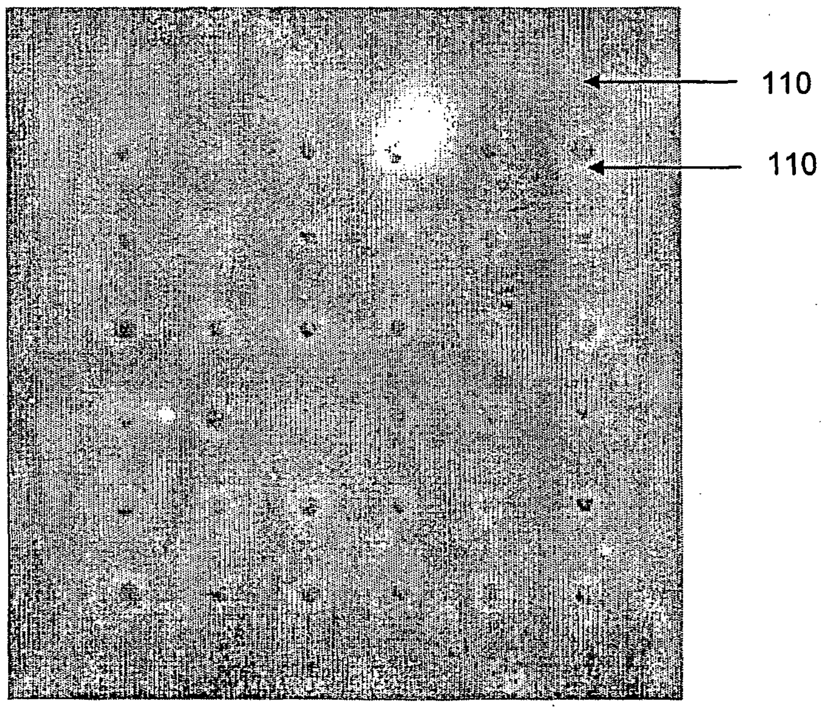


Figure 3B

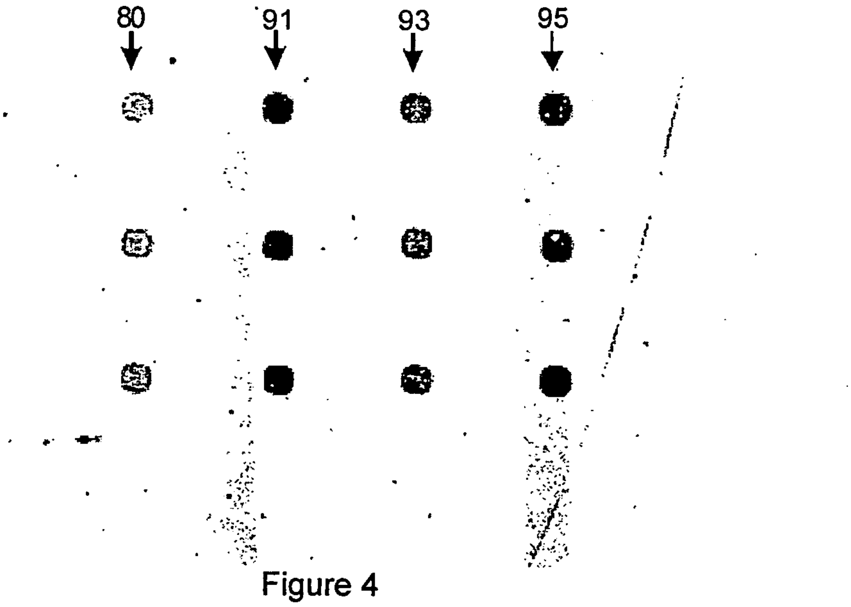


Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6740491 B
- US 6777186 B
- US 6773884 B
- US 6767702 B
- US 6759199 B
- US 6750016 B
- US 6730269 B
- US 6720411 B
- US 6720147 B
- US 6709825 B
- US 6682895 B
- US 6673548 B
- US 6667122 B
- US 6645721 B
- US 6610491 B
- US 6582921 B
- US 6506564 B
- US 6495324 B
- US 6417340 B
- US 6361944 B
- US 6602669 B
- US 5681702 A
- US 5597909 A
- US 5580731 A
- US 5359100 A
- US 5124246 A
- US 5545730 A
- US 5594117 A
- US 5571670 A
- US 5594118 A
- US 5681697 A
- US 5591584 A
- US 5624802 A
- US 5635352 A
- US 5710264 A
- US 5175270 A
- US 5487973 A
- US 5484904 A
- US 6593100 B
- US 6372937 B
- US 5185243 A
- US 5573907 A
- US 5011769 A
- US 5403711 A
- US 5660988 A
- US 4876187 A
- EP 0383126 A


### Non-patent literature cited in the description


- **Park, S.-J. et al.** *Science,* 2002, vol. 295 (5559), 1503-1506
- **Kern, D. ; Collins, M. ; Fultz, T. ; Detmer, J. ; Hamren, S. ; Peterkin, J. ; Sheridan, P. ; Urdea, M ; White, R. ; Yeghiazarian, T.** An Enhanced-sensitivity Branched-DNA Assay for Quantification of Human Immunodeficiency Virus Type 1 RNA in Plasma. *Journal of Clinical Microbiology,* 1996, vol. 34, 3196-3203
- **Stears, R. et al.** *Physiol. Genomics,* 2000, vol. 3, 93-99
- **Baner et al.** *Nuc. Acids Res.,* 1998, vol. 26, 5073-5078
- **Barany, F.** *Proc. Natl. Acad. Sci. USA,* 1991, vol. 88, 189-193
- **Lizardi et al.** *Nat. Genet.,* 1998, vol. 19, 225-232
- **Zhang et al.** *Gene,* 1998, vol. 211, 277
- **Daubendiek et al.** *Nature Biotech.,* 1997, vol. 15, 273
- **Nallur, G. ; Luo, C. ; Fang, L. ; Cooley, S. ; Dave, V. ; Lambert, J. ; Kukanskis, K. ; Kingsmore, S. ; Lasken, R. ; Schweitzer, B.** Signal Amplification by Rolling Circle Amplification on DNA Microarrays. *Nucleic Acids Research,* 2001, vol. 29, E118
- **Xu et al.** *Electoanalysis,* 2001, vol. 13 (10), 882-887
- **Biesalski, M. et al.** *J. Chem. Phys.,* 1999, vol. 111 (15), 7029
- **Husemann, M. et al.** *Agnewandte Chemie Int. Ed.,* 1999, vol. 38 (5), 647-649
- **Shah, R.R. et al.** *Macromolecules,* 2000, vol. 33, 597-605
- **Wang, D. ; Coscoy, L. ; Zylberberg, M. ; Avila, P.C. ; Boushey, H.A. ; Ganem, D. ; DeRisi, J.L.** Microarray Based Detection and Genotyping of Viral Pathogens. *PNAS,* 2002, vol. 99 (24), 15687-15692

- **Anthony, R.M. ; Brown, T.J. ; French, G.L.** Rapid Diagnosis of Bacteremia by Universal Amplification of 23S Ribosomal DNA Followed by Hybridization to an Oligonucleotide Array. *Journal of Clinical Microbiology,* 2000, vol. 38, 781-788
- **Risberg, E.** *Gene Chip Helps identify Cause of Mystery Illness,* 18 June 2003
- **Li, J. ; Chen, S. ; Evans, D.H.** Typing and Subtyping Influenza Virus Using DNA Microarrays and Multiplex Reverse Transcriptase PCR. *Journal of Clinical Microbiology,* 2001, vol. 39, 696-704
- **Schena, M.** Microarray Analysis. John Wiley & Sons, 2003, 153
- **Schena, M.** Microarray Analysis. John Wiley & Sons, 2003, 151
- **Uhlmann et al.** *Angew. Chem. Int. Ed. Eng.,* 1990, vol. 90, 543
- **Goodchild.** *J. Bioconjugate Chem.,* 1990, vol. I, 165
- **Englisch et al.** *Angew, Chem. Int. Ed. Eng.,* 1991, vol. 30, 613
- **Lesnik et al.** *Biochemistry,* 1993, vol. 32, 7832
- **Nielsen et al.** *Anti-Cancer Drug Design,* 1993, vol. 8, 53
- **Engels et al.** *Angew, Chem. Int. Ed. Eng.,* 1992, vol. 31, 1008
- **Burger, D.R.** *J. Clinical Immunoassay,* 1993, vol. 16, 224
- Protocols for Oligonucleotides and Analogs. **Uhlmann et al.** Methods in Molecular Biology. Humana Press, 1993, vol. 20, 335-389
- Methods in Molecular Biology. **Uhlmann et al.** Protocols for Oligonucleotides and Analogs. Humana Press, 1993, vol. 20, 335
- **Schena, M.** Microarray Analysis. John Wiley & Sons, 2003, 8
- **Constans, A.** *The Scientist,* 2004, vol. 18 (15), 42
- **Schena, M.** Microarray Analysis. John Wiley & Sons, 2003, 154
- **Dimmock, N.J. ; Easton, A.J. ; Leppard, K.N.** Introduction to Modern Virology. Blackwell Science Ltd, 2001
- **Lamb, R.A. ; Krug, R.M.** Orthomyxoviridae: The Viruses and their Replication, in Fields Virology. Lippincott-Raven, 1996
- **Li, J. et al.** *J. Clinical Microbio.,* 2001, vol. 39 (2), 696-704
- Development and application of nucleic acid probes. **Stahl, D. A. ; R. Amann.** Nucleic acid techniques in bacterial systematics. John Wiley & Sons Ltd, 1991, 205-248
- **Schena, M.** Microarray Analysis. John Wiley & Sons, 2003, 117
- Avidin-biotin technology. Methods in Enzymology. 1990, 184
- **Bhanu, V.A. ; Kishore, K.** Role of Oxygen in Polymerization Reactions. *Chemical Reviews,* 1991, vol. 91, 99-117
- **Hacioglu B. ; Berchtold K.A. ; Lovell L.G. ; Nie J. ; Bowman C.N.** Polymerization Kinetics of HEMA/DEGDMA: using Changes in initiation and Chain Transfer Rates to Explore the Effects of Chain-Length-Dependent Termination. *Biomaterials,* 2002, vol. 23, 4057-4064
- **Vernet, G.** DNA-Chip Technology and Infectious Diseases. *Virus Research,* 2002, vol. 82, 65-71
- **Wilbur, D.S. ; Hamlin, D.K. ; Buhler, K.R. ; Pathare, P.M. ; Vessella, R.L. ; Stayton, P.S. ; To, R.** Streptavidin in antibody pretargeting. 2. Evaluation of methods for decreasing localization of streptavidin to kidney while retaining its tumor binding capacity. *Bioconjugate Chemistry,* 1998, vol. 9, 322-330
- **Hermanson, G.T.** Bioconjugate Techniques. Academic Press, 1996, 435
- Protein biotinylation. Methods in Enzymology. 1990, vol. 184, 138-160
- **Kamigaito M. ; Ando T. ; Sawamoto M.** Metal-Catalyzed Living Radical polymerization. *Chemical Reviews,* 2001, vol. 101, 3689-3746
- **Matyjaszewski, K. ; Jianhui Xia, J.** *Atom Transfer Radical Polymerization Chemical Reviews,* 2001, vol. 101, 2921-2990